# EUROPEAN PATENT SPECIFICATION

(11) **EP 4 280 197 B1**
(45) Date of publication and mention of the grant of the patent: **26.11.2025**
(21) Application number: 22194201.4
(22) Date of filing: 06.09.2022
(51) Int. Cl.: G09B 19/00, A61B 5/11, B64C 39/02, G16H 20/30, G16H 30/20, G16H 30/40, G16H 50/20, G16H 40/63, B64U 101/05

(54) **TRAINING METHODS AND TRAINING SYSTEMS UTILIZING UNCREWED VEHICLES**
TRAININGSVERFAHREN UND TRAININGSSYSTEME MIT UNBEMANNTEN FAHRZEUGEN
PROCÉDÉS D'ENTRAÎNEMENT ET SYSTÈMES D'ENTRAÎNEMENT UTILISANT DES VÉHICULES SANS ÉQUIPAGE

(30) Priority: 20.05.2022 US 202217749695
(43) Date of publication of application: 22.11.2023
(73) Proprietor: National Cheng Kung University, Tainan City 701 (TW)
(72) Inventor: SU, FONG-CHIN, Tainan City 701 (TW); LIN, CHIEN-JU, Tainan City 700 (TW); CHIEH, HSIAO-FENG, Kaohsiung City 813 (TW)
(74) Representative: Yang, Shu

(56) References cited:
- WO-A1-2017/055080
- WO-A1-2017/074499
- US-A1- 2019 077 007
- US-A1- 2019 377 345
- US-A1- 2020 114 243
- US-A1- 2020 338 392
- LA DELFA JOSEPH CAFECIAOJOE@GMAIL COM ET AL: "Designing Drone Chi Unpacking the Thinking and Making of Somaesthetic Human-Drone Interaction", PROCEEDINGS OF THE 2020 ACM DESIGNING INTERACTIVE SYSTEMS CONFERENCE, ACMPUB27, NEW YORK, NY, USA, 3 July 2020 (2020-07-03), pages 575 - 586, XP058455177, ISBN: 978-1-4503-6974-9, DOI: 10.1145/3357236.3395589
- SERGEJ G ZWAAN ET AL: "Boxing against drones", INTERACTION DESIGN AND CHILDREN, ACM, 2 PENN PLAZA, SUITE 701 NEW YORK NY 10121-0701 USA, 21 June 2016 (2016-06-21), pages 607 - 612, XP058276621, ISBN: 978-1-4503-4313-8, DOI: 10.1145/2930674.2935991

## Description

### FIELD

The present disclosure generally relates to functional training and, more specifically, to training methods and training systems utilizing uncrewed vehicles.

### BACKGROUND

Functional training is a rehabilitation technique that mainly focuses on restoring strength and proper functions of the neuromusculoskeletal system, with the goal of making it easier for patients to perform their everyday activities. In other words, functional training is meant to improve the activities of daily living (ADL), such as reaching, showering, teeth brushing, housekeeping, etc.

Conventionally, guidance for performing functional training is provided through video or audio in order to instruct the patients to perform a sequence of actions. However, functional training should be different for everyone. For example, the effective range of motion for a 180 centimeter (cm) tall youth performing functional training should be greater than that for a 140 cm tall elder. Therefore, using audio or two-dimensional video as training guidance is insufficient.

LA DELFA JOSEPH CAFECIAOJOE@GMAIL COM ET AL, "Designing Drone Chi Unpacking the Thinking and Making of Somaesthetic Human-Drone Interaction", PROCEEDINGS OF THE 2020 ACM DESIGNING INTERACTIVE SYSTEMS CONFERENCE, ACMPUB27, NEW YORK, NY, USA, (20200703), doi:10.1145/3357236.3395589, ISBN 978-1-4503-6974-9, pages 575 - 586, XP058455177 [X] 1,11 * pages 575-585 * [I] 2-10 discloses Drone Chi, a Tai Chi inspired human-drone interaction experience. As a design research project, situated within somaesthetic interaction design, where a central topic is cultivating bodily and sensory appreciation to improve one's quality of life. Drone Chi investigates the potential of autonomous micro-quadcopters as a design material for somaesthetic HCI. Through a quasi-chronological account of the design process, this pictorial articulates how the sensory experiences of Tai Chi were integrated into Drone Chi. Taking a slow and open-ended design research approach, we iteratively developed the project through somaesthetic, product design and engineering perspectives and drew heavily on design analogies and imagery for inspiration. This elevated the influence of the soma amongst narrow engineering parameters and usability requirements. This pictorial serves as a reflective resource for designers who are experimenting with merging their native discipline with someasthetic interaction design.

WO2017/055080 A1 discloses a person performing physical exercises, comprising: - a flying, and/or ground-moving drone; - a projector arranged at the drone; - a surveillance sensor arranged at the drone for observing spatial confinements in an environment of the person and for identifying one or more surfaces in the environment of the person; - a memory unit for storing images or image sequences relating to training instructions for different kinds of physical exercises; and - a control unit which is configured: to select at least one of the one or more surfaces identified by the surveillance sensor, based on at least one of: (i) the observed spatial confinements, (ii) the kind of physical exercise, and (iii) characteristics of the one or more surfaces, and to control the projector upon request to project at least one of the images or image sequences of the at least one subset onto the at least one surface that has been selected for said at least one image or image sequence.

US2020/114243 A1 discloses a full scale practice, training and diagnostic system, method and software medium for providing highlighted progression and tempo illuminations of a practicing player in a first variant. Additional variants provide for field embedded pressure sensors in the field/court surface, as well as on the individual training or practicing person or player, in order to provide each of impact/injury analysis as well as multi-dimensional sensor profile analysis for a given practice activity.

WO2017/074499 A1 discloses a method for controlling a drone including performing operations on a processor configured to control location of the drone are described. The operations on the processor include receiving heart rate messages from a remote device carried by a user, where each heart rate message includes heart rate information of the user, and receiving location messages from the remote device carried by the user, where each location message includes location information of the user. The method includes predicting a future location of the user based on the heart rate messages and the location messages, generating a target location to which the drone is to be moved based on the future location of the user, and commanding the drone to move to the target location. Related devices are disclosed.

### SUMMARY

The present disclosure is directed to training methods and training systems utilizing uncrewed vehicles, which provide a more comprehensive and effective training.

According to a first aspect of the present disclosure, a training method performed by a training system including a plurality of uncrewed vehicles is provided in accordance with independent claim 1.

In an implementation of the first aspect of the present disclosure, controlling the corresponding uncrewed vehicle to provide the perceptible prompt includes suspending the movement of the corresponding uncrewed vehicle.

In another implementation of the first aspect of the present disclosure, in a case of determining that one of the plurality of body reactions matches the corresponding preset reaction, the method further includes continuing the movement of the corresponding uncrewed vehicle along one of the plurality of first preset trajectories of the corresponding uncrewed vehicle.

In another implementation of the first aspect of the present disclosure, controlling the corresponding uncrewed vehicle to provide the perceptible prompt includes controlling the movement of the plurality of uncrewed vehicles along a plurality of a second preset trajectories, wherein the plurality of second trajectories are different from the plurality of first preset trajectories.

In another implementation of the first aspect of the present disclosure, detecting the plurality of body reactions of the plurality of parts of the user's body includes obtaining location information of each of the plurality of parts of the user's body.

In another implementation of the first aspect of the present disclosure, determining whether the plurality of body reactions match the plurality of preset reactions corresponding to the plurality of first preset trajectories includes: determining, based on the location information, whether a distance between one of the plurality of uncrewed vehicles and one of the plurality of parts of the user's body is within a predetermined range; in a case that the distance is outside the predetermined range, determining that one of the plurality of the body reactions does not match one of the plurality of the preset reactions corresponding to the one of the plurality of first preset trajectories; and in a case that the distance is within the predetermined range, determining that one of the plurality of the body reactions matches one of the plurality of the preset reactions corresponding to one of the plurality of the first preset trajectories.

In another implementation of the first aspect of the present disclosure, determining whether the plurality of body reactions match the plurality of preset reactions corresponding to the plurality of first preset trajectories includes: determining, based on the location information, whether a distance between one of the uncrewed vehicles and one of the plurality of parts of the user's body is greater than a maximum threshold; in a case that the distance is greater than the maximum threshold, determining that one of the plurality of the body reactions does not match one of the plurality of the preset reactions corresponding to one of the plurality of the first preset trajectories; and in a case that the distance is not greater than the maximum threshold, determining that one of the plurality of the body reactions matches one of the plurality of the preset reactions corresponding to one of the plurality of the first preset trajectories.

In another implementation of the first aspect of the present disclosure, determining whether the plurality of body reactions match the plurality of preset reactions corresponding to the plurality of first preset trajectories includes: determining, based on the location information, whether a distance between one of the plurality of uncrewed vehicles and one of the plurality of parts of the user's body is less than a minimum threshold; in a case that the distance is less than the minimum threshold, determining that one of the plurality of the body reactions does not match one of the plurality of the preset reactions corresponding to one of the plurality of the first preset trajectories; and in a case that the distance is not less than the minimum threshold, determining that one of the plurality of the body reactions matches one of the plurality of the preset reactions corresponding to one of the plurality of the first preset trajectories.

In another implementation of the first aspect of the present disclosure, controlling the plurality of the uncrewed vehicles to provide the perceptible prompt includes controlling the plurality of the uncrewed vehicle to move away from the plurality of parts of the user's body.

In another implementation of the first aspect of the present disclosure, detecting the plurality of body reactions of the plurality of parts of the user's body includes obtaining muscle strength information of the plurality of parts of the user's body, and the muscle strength information includes at least one of a strength magnitude and a strength direction.

According to a second aspect of the present disclosure, a training system is provided according to independent claim 11.

According to the above, the training method of the present disclosure provides motion guidance in a three-dimensional real world, which not only brings challenges to different users according to their needs, but also involves visual feedback in the functional training. Hence, a more comprehensive and effective training is achieved.

### BRIEF DESCRIPTION OF THE DRAWINGS

Aspects of the present disclosure are best understood from the following detailed description when read with the accompanying figures. Various features are not drawn to scale. Dimensions of various features may be arbitrarily increased or reduced for clarity of discussion.
FIG. 1 is a block diagram illustrating a training system, according to an example implementation of the present disclosure.
FIG. 2 is a diagram illustrating a training system in operation, according to an example implementation of the present disclosure.
FIG. 3A, FIG. 3B, FIG. 3C, and FIG. 3D are block diagrams illustrating various arrangements of the training system, according to an example implementation of the present disclosure.
FIG. 4 is a flowchart illustrating a training method, according to an example implementation of the present disclosure.
FIG. 5 is a timing diagram illustrating a distance between the uncrewed vehicle and a part of the user's body, according to an example implementation of the present disclosure.

### DESCRIPTION

The following contains specific information pertaining to example implementations in the present disclosure. The drawings and their accompanying detailed disclosure are directed to merely example implementations of the present disclosure. However, the present disclosure is not limited to merely these example implementations. Other variations and implementations of the present disclosure will occur to those skilled in the art. Unless noted otherwise, like or corresponding elements among the figures may be indicated by like or corresponding reference numerals. Moreover, the drawings and illustrations in the present disclosure are generally not to scale and are not intended to correspond to actual relative dimensions.

For consistency and ease of understanding, like features are identified (although, in some examples, not illustrated) by numerals in the example figures. However, the features in different implementations may differ in other respects, and thus shall not be narrowly confined to what is illustrated in the figures.

References to "one implementation," "an implementation," "example implementation," "various implementations," "some implementations," "implementations of the present disclosure," etc., may indicate that the implementation(s) of the present disclosure may include a particular feature, structure, or characteristic, but not every possible implementation of the present disclosure necessarily includes the particular feature, structure, or characteristic. Further, repeated use of the phrase "in one implementation," "in an example implementation," or "an implementation," do not necessarily refer to the same implementation, although they may. Moreover, any use of phrases like "implementations" in connection with "the present disclosure" are never meant to characterize that all implementations of the present disclosure must include the particular feature, structure, or characteristic, and should instead be understood to mean "at least some implementations of the present disclosure" include the stated particular feature, structure, or characteristic. The term "coupled" is defined as connected, whether directly or indirectly through intervening components, and is not necessarily limited to physical connections. The term "comprising," when utilized, means "including but not necessarily limited to"; it specifically indicates open-ended inclusion or membership in the disclosed combination, group, series, and the equivalent.

The term "and/or" herein is only an association relationship for describing associated objects and represents that three relationships may exist; for example, A and/or B may represent that: A exists alone, A and B exist at the same time, and B exists alone. "A and/or B and/or C" may represent that at least one of A, B, and C exists. The character "/" used herein generally represents that the former and latter associated objects are in an "or" relationship.

Additionally, for a non-limiting explanation, specific details, such as functional entities, techniques, and the like, are set forth for providing an understanding of the disclosed technology. In other examples, detailed disclosure of well-known methods, technologies, systems, architectures, and the like are omitted so as not to obscure the present disclosure with unnecessary details.

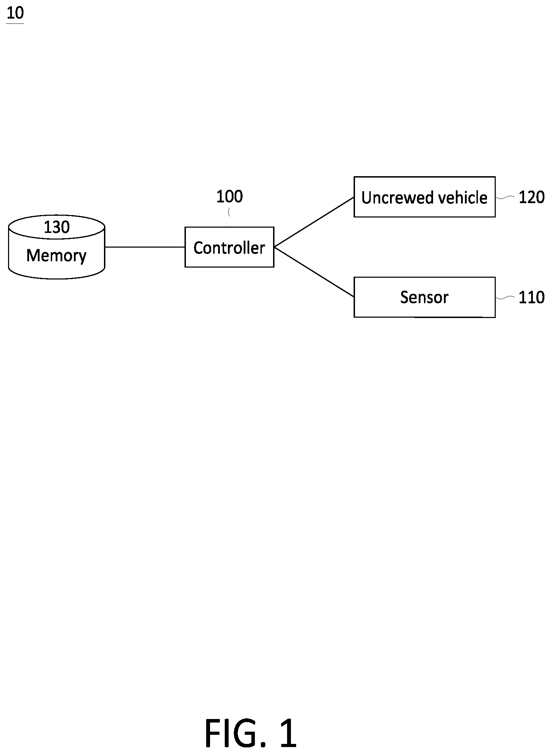

FIG. 1 is a block diagram illustrating a training system according to an example implementation of the present disclosure.

Referring to FIG. 1, training system 10 includes a controller 100, a sensor 110, an uncrewed vehicle 120, and a memory 130, where the controller 100 is coupled, by wire and/or wirelessly, to the sensor 110, the uncrewed vehicle 120, and the memory 130. The training system 10 is an interactive training system which is capable of guiding a user to perform a sequence of training actions by using the uncrewed vehicle moving in a three-dimensional (3D) real world and providing feedback when the user does not follow the provided guidance. The sequence of training actions may be, for example, a "Baduanjin qigong", which includes eight separate exercises each focusing on a different physical area, and "qi meridian", but is not limited thereto.

It is noted that, although the training system 10 in the following implementations includes one controller 100, one sensor 110, one uncrewed vehicle 120, and one memory 130 for exemplary illustration, the number of controllers 100, the number of sensors 110, the number of uncrewed vehicles 120, and the number of memories 130 in the training system 10 are not limited in the present disclosure. The training system 10 includes multiple uncrewed vehicles 120, each configured to guide a body segment of the user.

The controller 100 is configured to access and execute the computer-executable instructions stored in the memory 130 to perform the training method of the present disclosure. In some implementations, the controller 100 may include a central processing unit (CPU) or another programmable general-purpose or special-purpose microprocessor, a digital signal processor (DSP), a programmable controller, an application-specific integrated circuit (ASIC), a programmable logic device (PLD), or other similar components or a combination of the components.

The sensor 110 is configured to detect a body reaction of a user's body and provide information of the detected body reaction to the controller 100. The body reaction is physiological feedback from the user in response to the guidance of the training system 10, which may be, for example, a movement of at least one part of the user's body (e.g., a body segment), a contraction of at least one muscle of the user, or a combination thereof. In some implementations, the sensor 110 may include, for example, one or more proximity sensors, a Loco positioning system (LPS) constructed by multiple ultra-wideband radio sensors, one or more cameras, one or more millimeter wave radar units, one or more electromyography (EMG) detectors, one or more force sensors, and/or other similar components or a combination of the components.

In an example, the proximity sensor may be mounted on an uncrewed vehicle 120 to obtain a distance between the uncrewed vehicle 120 and a part of the user's body.

In an example, the LPS may include at least one anchor and multiple tags (e.g., placed on the uncrewed vehicle and a part of the user's body) and be configured to record the location of each tag in the three-dimensional (3D) space established by the LPS (e.g., relative to the at least one anchor). In the LPS, the anchor(s) acts as a signal transmitter and the tag(s) acts as a signal receiver, but the LPS is not limited to such an implementation.

In an example, one camera may be configured to obtain an image of the user and the image may be analyzed by the controller 100 performing image processing to obtain the body reaction, such as movements of the skeleton, orientations of the face, etc., of the user. On the other hand, more than one camera may be further configured to construct a 3D space. Through the image processing, locations of the uncrewed vehicle 120 and body segments of the user in the 3D space may be obtained.

The millimeter wave radar may detect distance, velocity, and trajectory of a target. In an example, the millimeter wave radar may be mounted on the uncrewed vehicle 120 and configured to detect dynamic locations and velocities of the user. By analyzing data of the millimeter wave radar, the controller 100 may obtain a relative distance between the uncrewed vehicle 120 and any body segment of the user.

In an example, each EMG detector may be mounted on a body segment of the user and configured to detect the muscle contraction of each body segment.

In an example, at least one (e.g., 6) force sensor(s) may be disposed on at least one finger(s) of the user and configured to detect the force exerted by the user's finger(s).

It is noted that the type of the sensor 110 is not limited to the above examples. One of ordinary skill in the art may design the sensor 120 in order to detect the body reaction of the user as their need.

The uncrewed vehicle 120 is configured to receive a command from the controller 100 and move, according to the received command, in the 3D real world to guide the user to perform a sequence of actions. The uncrewed vehicle 120 may be, for example, an unmanned aerial vehicle (UAV, also referred to as a drone), or an unmanned ground vehicle.

In some implementations, one uncrewed vehicle 120 may be configured to guide multiple body segments of the user. For example, the uncrewed vehicle may move along a 1-shape trajectory in the air to guide the user to perform the first section or the first exercise of the "Baduanjin qigong"; and move along a 2-shape trajectory in the air to guide the user to perform the second section or the second exercise of the "Baduanjin qigong"; and so on.

In some implementations, one uncrewed vehicle 120 may be configured to guide one body segment of the user. For example, the training system 10 may include 4 uncrewed vehicles for guiding arms and legs of the user. However, the number of uncrewed vehicles 120 in the training system 10 is not limited in the present disclosure. In some implementations, the training system 10 may include 10 uncrewed vehicles for guiding the head, the upper trunk, the left arm, the left forearm, the right arm, the right forearm, the left thigh, the left shank, the right thigh, and the right shank of the user, respectively.

The memory 130 is configured to store at least one preset trajectory and at least one computer-executable instruction. The memory 130 may include, for example, Random Access Memory (RAM), Read-Only Memory (ROM), Erasable Programmable Read-Only Memory (EPROM), Electrically Erasable Programmable Read-Only Memory (EEPROM), flash memory, Compact Disc Read-Only Memory (CD-ROM), magnetic cassettes, magnetic tape, magnetic disk storage, or any other equivalent medium capable of storing computer-executable instructions.

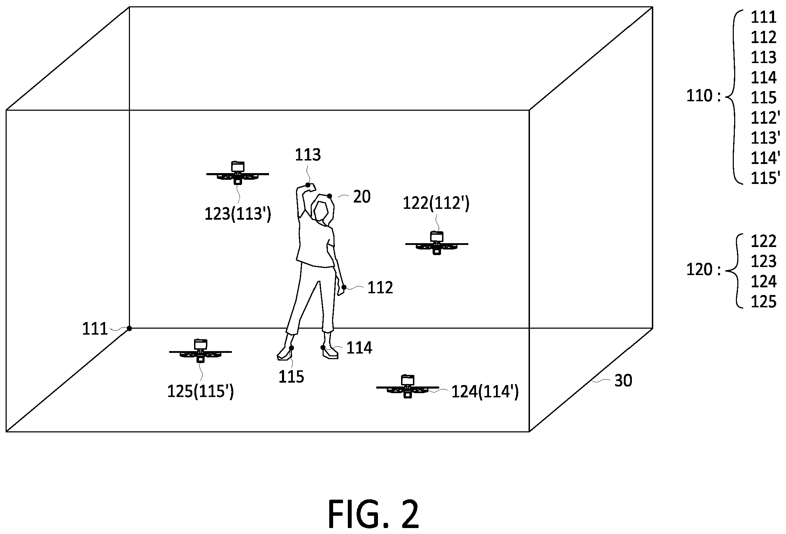

FIG. 2 is a diagram illustrating a training system in operation according to an example implementation of the present disclosure. In the example implementation of FIG. 2, an LPS is taken as an example of the sensor 110, and four UAVs 122, 123, 124, and 125 are included in the training system 10 for respectively guiding two arms and two legs of the user 20.

Referring to FIG. 2, in some implementations, the sensor 110 of the training system 10 is the LPS and includes an anchor 111 and multiple tags 112, 113, 114, 115, 112', 113', 114', and 115'. The LPS system defines a 3D coordinate system 30 where the anchor 111 is located at the origin (0, 0, 0), for example.

The tag 112 is placed at the left wrist of the user 20, the tag 113 is placed at the right wrist of the user 20, the tag 114 is placed at the left ankle of the user 20, and the tag 115 is placed at the right ankle of the user 20. In this case, the LPS system may obtain the locations (Xlw, Ylw, Zlw), (Xrw, Yrw, Zrw), (Xla, Yla, Zla), and (Xra, Yra, Zra) of each tag 112, 113, 114, and 115, respectively, over time in the 3D coordinate system 30, where the locations (Xlw, Ylw, Zlw), (Xrw, Yrw, Zrw), (Xla, Yla, Zla), and (Xra, Yra, Zra) of each tag 112, 113, 114, and 115 may represent the locations or positions of the left arm, the right arm, the left leg, and the right leg, respectively, of the user 20.

The tag 112' is placed at the UAV 122 configured to guide the left arm of the user 20, the tag 113' is placed at the UAV 123 configured to guide the right arm of the user 20, the tag 114' is placed at the UAV 124 configured to guide the left leg of the user 20, and the tag 115' is placed at the UAV 125 configured to guide the right leg of the user 20. In this case, the LPS system may obtain the locations (Xlw', Ylw', Zlw'), (Xrw', Yrw', Zrw'), (Xla', Yla', Zla'), and (Xra', Yra', Zra') of each tag 112', 113', 114', and 115', respectively, in the 3D coordinate system 30 over time, where the locations (Xlw', Ylw', Zlw'), (Xrw', Yrw', Zrw'), (Xla', Yla', Zla'), and (Xra', Yra', Zra') of each tag 112', 113', 114', and 115' may represent the locations or positions of the UAVs 122, 123, 124, and 125, respectively.

In the training system 10 of FIG. 2, the sensor 110 and the uncrewed vehicles 120 are separate devices, and the controller 100 (not shown in FIG. 2) may be, for example, separate from the sensor 110 and the uncrewed vehicles 120, integrated with the sensor 110, or integrated with one or each of the uncrewed vehicles 120. In other words, the arrangement of the components in the training system 10 is not limited in the present disclosure. Various implementations of the arrangements of the training system 10 are illustrated in the following description.

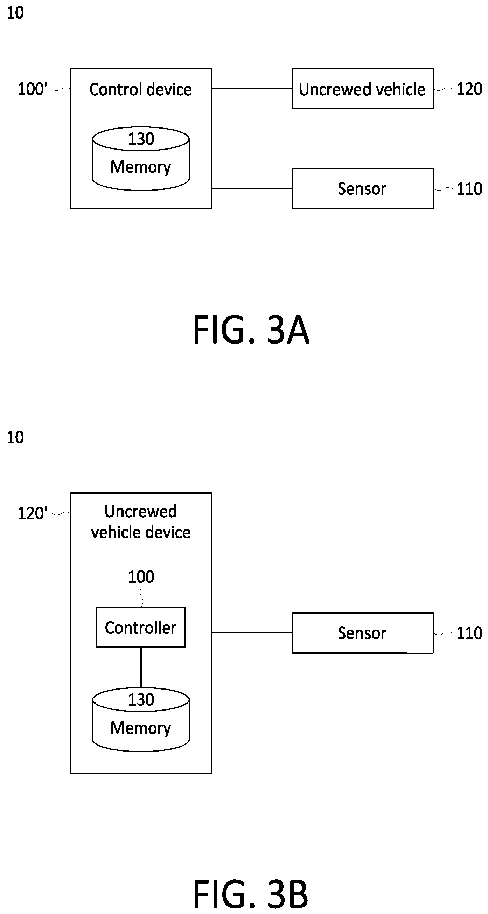

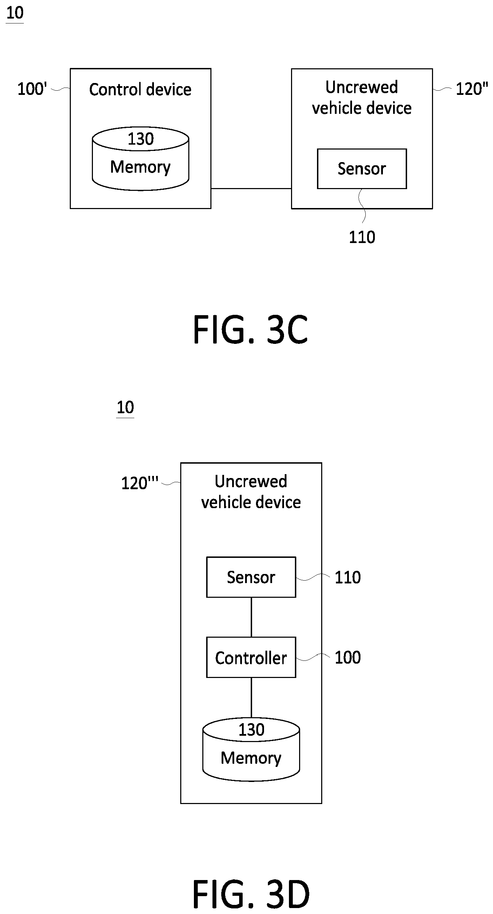

FIG. 3A, FIG. 3B, FIG. 3C, and FIG. 3D are block diagrams illustrating various arrangements of the training system according to an example implementation of the present disclosure.

Referring to FIG. 3A, in some implementations, the controller 100 and the memory 130 may be integrated as a control device 100', and the control device 100', the sensor 110, and the uncrewed vehicle 120 are separate devices.

Taking FIG. 2 as an example, the sensor 110 and the uncrewed vehicles 120 may be separate devices, and the control device 100' may be separate from the sensor 110 and the uncrewed vehicles 120 as well. The control device 100' may, for example, locate at the origin (0, 0, 0), receive the location information from the sensor 110, and control the uncrewed vehicles 120.

In some examples, the control device 100' is configured to receive location information (e.g., the locations in the 3D coordinate system 30) from the sensor 110 and control the uncrewed vehicle(s) 120.

Referring to FIG. 3B, in some implementations, the control device 100' may be further integrated into uncrewed vehicle 120 as an uncrewed vehicle device 120', and the uncrewed vehicle device 120' is separate from the sensor 110.

Taking FIG. 2 as an example, the control device 100' (e.g., the controller 100 and the memory 130) may be integrated into one of the uncrewed vehicles 122, 123, 124, or 125 as the uncrewed vehicle device 120', and the control device 100' in the uncrewed vehicle device 120' is wirelessly coupled to the sensor 110 (e.g., the LPS) and the other uncrewed vehicles 120.

In some examples, the control device 100' is configured to receive location information (e.g., the locations in the 3D coordinate system 30) from the sensor 110 and control the uncrewed vehicle device 120' and the other uncrewed vehicle(s) 120.

Taking FIG. 2 as another example, four control devices 100' may be respectively integrated into the uncrewed vehicles 122, 123, 124, and 125 as four uncrewed vehicle devices 120', and the four control devices 100' are wirelessly coupled to each other and the sensor 110 (e.g., the LPS).

In some examples, each control device 100' is configured to receive location information (e.g., the locations in the 3D coordinate system 30) from the sensor 110 and control the respective uncrewed vehicle device(s) 120'.

Referring to FIG. 3C, in some implementations, the controller 100 and the memory 130 may be integrated as a control device 100', and the uncrewed vehicle 120 and the sensor 110 may be integrated as an uncrewed vehicle device 120".

For example, four sensors 110 each corresponding to the left arm, the right arm, the left leg, and the right leg of the user 20 may by respectively disposed on the uncrewed vehicle 120 guiding the left arm, the uncrewed vehicle 120 guiding the right arm, the uncrewed vehicle 120 guiding the left leg, and the uncrewed vehicle 120 guiding the right leg. The sensor 110 disposed on the uncrewed vehicle 120 guiding the left arm of the user 20 is configured to detect a distance to the left arm of the user 20, the sensor 110 disposed on the uncrewed vehicle 120 guiding the right arm of the user 20 is configured to detect a distance to the right arm of the user 20, the sensor 110 disposed on the uncrewed vehicle 120 guiding the left leg of the user 20 is configured to detect a distance to the left leg of the user 20, and the sensor 110 disposed on the uncrewed vehicle 120 guiding the right leg of the user 20 is configured to detect a distance to the right leg of the user 20.

In some examples, the control device 100' is separated from the four uncrewed vehicle device 120" and configured to receive location information (e.g., the detected distances) from the sensor(s) 110 and control the uncrewed vehicle device(s) 120".

However, the implementation for detecting the distance is not limited in the present disclosure. In some examples, the sensor 110 may be a proximity sensor or a millimeter wave radar disposed on each uncrewed vehicle 120 for detecting the distance to a corresponding part of the user's body, and the sensor 110 is not limited to such implementations in the present disclosure.

Referring to FIG. 3D, in some implementations, the controller 100, the sensor 110, and the memory 130 may be integrated into the uncrewed vehicle 120 as an uncrewed vehicle device 120‴.

For example, four uncrewed vehicle devices 120‴ may be included in the training system 10, and each of the four uncrewed vehicle devices 120‴ may include a controller 100, a sensor 110, and a memory 130. In this case, the four uncrewed vehicle devices 120‴ are configured to respectively guide four parts of the user's body. The four controllers 100 are wirelessly coupled to each other in order to communicate with each other.

In some examples, each controller 100 is configured to receive location information (e.g., the distance as detected in FIG. 3C) from the sensor 110 and control the respective uncrewed vehicle device(s) 120‴.

Although four uncrewed vehicles are illustrated in the training system 10 in the above implementations, it should be noted that the number of uncrewed vehicles 120 is not limited to four. Alternatively, the number of uncrewed vehicles 120 in the training system 10 may be two, three, or more than four.

In addition, although four exemplary arrangements of the training system 10 are illustrated with reference to FIG. 3A, FIG. 3B, FIG. 3C, and FIG. 3D, it should be noted that the arrangement of the components in the training system 10 is not limited to the four illustrated arrangements. One of ordinary skill in the art may implement the training system 10 according to their needs.

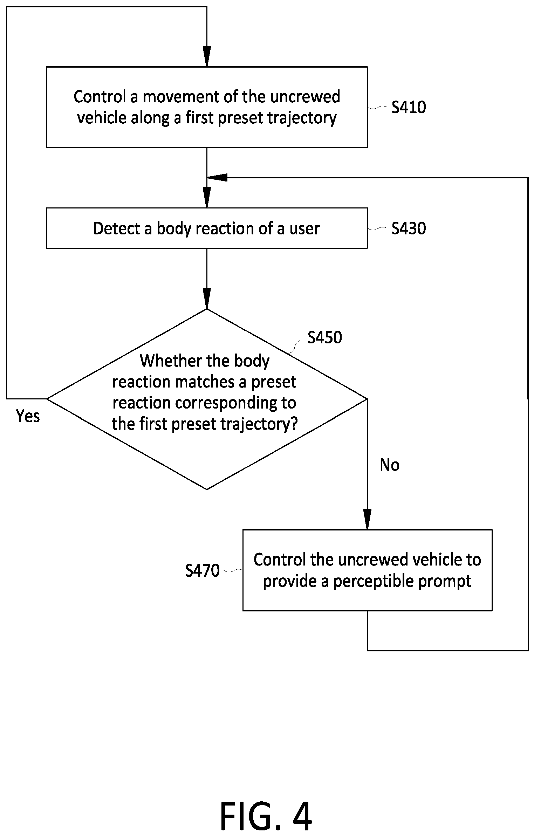

FIG. 4 is a flowchart illustrating a training method according to an example implementation of the present disclosure. The training method illustrated in FIG. 4 may be performed by the training system 10 described above; therefore, the same reference numerals will be used for describing the actions of the training method illustrated in FIG. 4 for convenience.

It is noted that although actions S410, S430, S450, and S470 are illustrated as separate actions represented as independent blocks, these separately illustrated actions should not be construed as necessarily order dependent. The order in which the actions are performed in FIG. 4 is not intended to be construed as a limitation, and any number of the disclosed blocks may be combined in any order to implement the method, or an alternate method.

Referring to FIG. 4, in action S410, the controller 100 may control a movement of the uncrewed vehicle 120 along a first preset trajectory. Specifically, the first preset trajectory is designed for guiding the user 20 to make a sequence of training actions, and the controller 100 may be configured to control the uncrewed vehicle to move along the first preset trajectory.

In some implementations, the first preset trajectory may, for example, include information of the location and the velocity over time (e.g., at each time point). In some implementations, the first preset trajectory may be, for example, set according to a length of a body segment of the user 20 and an expected completion time of each move. As such, the guidance provided by the uncrewed vehicle 120 moving along the first preset trajectory can meet the needs of different users.

In some implementations, only one uncrewed vehicle 120 is included in the training system 10. The uncrewed vehicle 120 moves along the first preset trajectory to guide the user to make expected movements of a training sequence. For example, the first preset trajectory may include a sequence of a 1-shape trajectory to an 8-shape trajectory for guiding the user 20 to sequentially execute eight exercises of the "Baduanjin qigong". For another example, the first preset trajectory may repeatedly go up and down in the air and the uncrewed vehicle 120 (e.g., UAV) may move (e.g., fly) along the first preset trajectory to lead a part of the user's body (e.g., left hand of the user 20 holding a dumbbell, or muscle(s) controlling the finger(s) of the user 20).

In some implementations, four UAVs 122, 123, 124, and 125 (e.g., as shown in FIG. 2) are respectively controlled by the controller(s) 100 to fly along four (different) first preset trajectories so as to guide the user 20 to perform the "Baduanjin qigong". For example, the UAV 122 flies along a first trajectory to guide the left arm of the user 20 to make the movement of the left arm in the Baduanjin qigong, the UAV 123 flies along a second trajectory to guide the right arm of the user 20 to make the movement of the right arm in the Baduanjin qigong, the UAV 124 flies along a third trajectory to guide the left leg of the user 20 to make the movement of the left leg in the Baduanjin qigong, and the UAV 125 flies along a fourth trajectory to guide the right leg of the user 20 to make the movement of the right leg in the Baduanjin qigong.

In some implementations, the training system 10 may further include a speaker. While controlling the movement of the uncrewed vehicle 120, the controller 100 may synchronously play a soundtrack (e.g., voice guidance or music of the "Baduanjin qigong") corresponding to the first preset trajectory.

Referring to FIG. 4, in action S430, the sensor 110 may detect a body reaction of the user 20, and in action S450, the controller 100 may determine whether the body reaction matches a preset reaction corresponding to the first preset trajectory. It is noted that the action S430 and the action S450 may be performed while the training system 10 is activated. That is, the actions S410, S430, and S450 may be, for example, performed simultaneously.

Specifically, at least a part of the user's body may react in response to the guidance provided by the uncrewed vehicle(s) 120, and the sensor 110 may detect the body reaction of the user 20 in response to the guidance provided by the uncrewed vehicle(s) 120. The body reaction may be, for example, associated with a muscular system or a nervous system of the user 20.

In some implementations, each of the first preset trajectory may correspond to a preset reaction.

In some implementations, the first preset trajectory may include a sequence of a 1-shape trajectory to an 8-shape trajectory for guiding the user 20 to sequentially execute eight exercises of the "Baduanjin qigong". In this case, the preset reaction may be, for example, the user 20 sequentially executing the eight exercises of the "Baduanjin qigong". For example, the user 20 should start to execute the first exercise in response to the uncrewed vehicle 120 finishing the 1-shape trajectory, and complete the first exercise before the uncrewed vehicle 120 starts to fly along the 2-shape trajectory; the user 20 should start to execute the second exercise in response to the uncrewed vehicle 120 finishing the 2-shape trajectory, and complete the second exercise before the uncrewed vehicle 120 starts to fly along the 3-shape trajectory; and so on.

In some implementations, the sensor 100 may obtain the image of the user 20, and the controller 100 may determine whether the user 20 is sequentially executing the eight exercises of the "Baduanjin qigong" by following the guidance provided by the uncrewed vehicle 120 (e.g., by image processing using an artificial intelligence (AI) model, etc.) In a case that the result of the image processing shows that the user 20 is sequentially executing the eight exercises of the "Baduanjin qigong", the controller 100 may determine that the body reaction matches the preset reaction. Otherwise, the controller 100 may determine that the body reaction does not match the preset reaction. One of ordinary skill in the art may implement the determination according to their needs; therefore, details of the determination are not described herein.

In some implementations, the first preset trajectory may repeatedly go up and down for leading a part of the user's body (for example, the left hand, but not limited thereto). In this case, the preset reaction may be, for example, the part of the user's body repeatedly raising up and lowering down by following the uncrewed vehicle 120.

In some implementations, the first preset trajectory may be a regular or irregular trajectory in the air for leading the part of the user's body (for example, the left, but not limited thereto). In this case, the preset reaction may be, for example, the part of the user's body moving along the regular or irregular trajectory in the air by following the first preset trajectory.

In some implementations, the sensor 100 may obtain the image of the user 20, and the controller 100 may determine whether the body reaction of the user 20 matches the preset reaction based on the image (e.g., by image processing using OpenCV^{™}, an open-source computer vision artificial intelligence (AI) model, etc.). In a case that the result of the image processing shows that the part of the user's body does follow the uncrewed vehicle 120 moving along the first preset trajectory, the controller 100 may determine that the body reaction matches the preset reaction. Otherwise, the controller 100 may determine that the body reaction does not match the preset reaction. One of ordinary skill in the art may implement the determination according to their needs; therefore, details of the determination are not described herein.

In some implementations, the sensor 100 may, for example, obtain a distance between the uncrewed vehicle 120 and the part of the user's body to determine whether the body reaction of the user 20 matches the preset reaction. For example, the distance may be a difference of the locations of the unmanned vehicle 120 and the part of the user's body in an established 3D coordinate system 30 (e.g., the distance between two locations of (Xlw, Ylw, Zlw) and (Xlw', Ylw', Zlw')). In another example, the distance may be a relative distance obtained directly by the sensor 110 mounted on the unmanned vehicle 120.

In some implementation, in a case that the distance between the uncrewed vehicle 120 and the part of the user's body is not greater than a maximum threshold, the controller 100 may determine that the body reaction matches the preset reaction; in a case that the distance between the uncrewed vehicle 120 and the part of the user's body is greater than the maximum threshold, the controller 100 may determine that the body reaction does not match the preset reaction.

In some implementation, in a case that the distance between the uncrewed vehicle 120 and the part of the user's body is not less than a minimum threshold, the controller 100 may determine that the body reaction matches the preset reaction; in a case that the distance between the uncrewed vehicle 120 and the part of the user's body is less than the minimum threshold, the controller 100 may determine that the body reaction does not match the preset reaction.

In some implementation, in a case that the distance between the uncrewed vehicle 120 and the part of the user's body is within a predetermined range, the controller 100 may determine that the body reaction matches the preset reaction; in a case that the distance between the uncrewed vehicle 120 and the part of the user's body is not within the predetermined range, the controller 100 may determine that the body reaction does not match the preset reaction. The predetermined range, for example, may be defined by the maximum threshold and the minimum threshold described above, but is not limited thereto.

In some implementations, each of the multiple uncrewed vehicles 120 moves along a different first preset trajectory to guide the corresponding part of the user's body. The controller 100 may determine whether each part of the user's body does follow the corresponding uncrewed vehicle 120. In a case that one of the uncrewed vehicles 120 is not followed (e.g., the distance between the one of the uncrewed vehicles 120 and the corresponding part of the user's body is greater than the maximum threshold, less than the minimum threshold, or not within the predetermined range), the controller 100 may determine that the body reaction of the user 20 does not match the preset reaction. Otherwise, the controller 100 may determine that the body reaction of the user 20 matches the preset reaction.

In some implementations, the first preset trajectory may repeatedly go left and right for guiding a part of the user's body (for example, the face, but not limited thereto). In this case, the preset reaction may be, for example, the orientation of the user's face repeatedly turns left and right by following the uncrewed vehicle 120.

In some implementations, the sensor 100 may obtain the image of the user 20, and the controller 100 may determine whether the body reaction of the user 20 matches the preset reaction based on the image (e.g., by image processing using OpenCV^{™}, etc.). In a case that the result of the image processing shows that the orientation of the user's face does follow the uncrewed vehicle 120 moving along the first preset trajectory, the controller 100 may determine that the body reaction matches the preset reaction. Otherwise, the controller 100 may determine that the body reaction does not match the preset reaction. One of ordinary skill in the art may implement the determination as to their needs; therefore, details of the determination are not described herein.

In some implementations, the first preset trajectory may be a regular or an irregular trajectory in the air for guiding the part of the user's body (for example, specific muscles controlling finger(s) of the user 20, but not limited thereto). In this case, the preset reaction may be, for example, the part of the user's body (for example, specific muscles controlling finger(s) of the user 20, but not limited thereto) contracting in response to the direction and the velocity of the first preset trajectory. For example, the specific muscles controlling the fingers of the user 20 may contract to lift the fingers up or apply an upward force when the uncrewed vehicle 120 goes up, and the specific muscles controlling the fingers of the user 20 may contract to bend the fingers down or apply a downward force when the uncrewed vehicle 120 goes down. In addition, the strength of the contraction may be, for example, positively related to the velocity of the uncrewed vehicle 120.

In some implementations, the sensor 110 may be an EMG detector or at least one (e.g., 6) force sensor(s), which is capable of detecting the body reactions such as muscle contractions or force exertions of the finger(s), even if the fingers do not actually bend in appearance. According to the muscle contractions and/or the force exertions detected by the sensor 110, the controller 100 may determine whether the body reaction matches the preset reaction.

Referring to FIG. 4, in a case that the controller 100 determines that the body reaction of the user 20 matches the preset reaction, the process goes back to action S410, which means that the uncrewed vehicle 120 keeps moving along the first preset trajectory without being affected by the actions of S430 and S450. In a case that the controller 100 determines that the body reaction of the user 20 does not match the preset reaction, the process goes to action S470, the controller 100 may control the uncrewed vehicle 120 to provide a perceptible prompt, then the process goes to action S430. It is noted that the action S430 and the action S450 may be performed while the perceptible prompt is provided. That is, the actions S430, S450, and S470 may be, for example, performed simultaneously.

Specifically, the perceptible prompt may be feedback to the user 20 for notifying that at least one part of the user 20 is not following the guidance of the uncrewed vehicle 120.

In some implementations, the perceptible prompt may include sound feedback. For example, the sound feedback may include an alarm, a preset soundtrack, and/or pausing the playing soundtrack (e.g., voice guidance or music of the "Baduanjin qigong",) etc., and is not limited to such forms in the present disclosure.

In some implementations, the perceptible prompt may include visual feedback. For example, the visual feedback may include a lighting effect, and/or a movement of the uncrewed vehicle 120 deviating from the first preset trajectory, etc., and is not limited to such forms in the present disclosure.

In some implementations, the perceptible prompt may include suspending the movement of the uncrewed vehicle 120. For example, the uncrewed vehicle 120 may move along the first preset trajectory for guiding the user 20 in the action S410. In a case that the controller 100 determines that the body reaction does not match the preset reaction, the movement of the uncrewed vehicle 120 (e.g., along the first preset trajectory) may be suspended until the controller 100 determines that the body reaction matches the preset reaction in the action S450. Once the controller 100 determines that the body reaction matches the preset reaction in the action S450, the uncrewed vehicle 120 may continue to move along the first preset trajectory for continuing the guidance to the user 20.

In some implementations, the perceptible prompt may further include the uncrewed vehicle 120 moving along a second preset trajectory. For example, in a case that the movement along the first preset trajectory is suspended, the uncrewed vehicle 120 may not need to stay in place. Instead, the controller 100 may control the uncrewed vehicle 120 to move along a second preset trajectory which is different from the first preset trajectory, in order to provide visual feedback to the user 20. The second preset trajectory may be, for example but not limited to, a circular trajectory or an 8-shape trajectory. Once the controller 100 determines that the body reaction matches the preset reaction in the action S450, the uncrewed vehicle 120 may continue to move along the first preset trajectory for continuing the guidance to the user 20. It should be noted that in this case, the controller 100 determines whether the body reaction matches the preset reaction by comparing the distance between the part of the user's body and the suspension point of the uncrewed vehicle 120, instead of the distance between the part of the user's body and the current location of the uncrewed vehicle 120, since the uncrewed vehicle 120 is moving to provide the perceptible prompt (e.g., visual feedback) instead of the training guidance.

In some implementations, the perceptible prompt may include moving away from the part of the user's body. In some cases, the controller 100 may determine that the body reaction does not match the preset reaction on the basis of the distance between the uncrewed vehicle 120 and the part of the user's body is less than the minimum threshold. Therefore, the controller 100 may control the uncrewed vehicle 120 to move away from the part of the user's body (e.g., move in an opposite direction to the movement of the part of the user's body) in order to avoid an occurrence of collision. It is noted that the actions S430 and S450 are performed while the perceptible prompt is provided. Once the controller 100 determines that the body reaction matches the preset reaction in the action S450 (e.g., the distance between the uncrewed vehicle 120 and the part of the user's body is not less than the minimum threshold), the uncrewed vehicle 120 may continue to move along the first preset trajectory for continuing the guidance to the user 20.

In some implementations, different perceptible prompts (e.g., different colors of light flashing on the uncrewed vehicle 120, different alarms sounding, or different second preset trajectories, etc.) may send different messages to the user. For example, a first perceptible prompt may send a message to the user 20 that the part of the user's body is too far from the uncrewed vehicle 120, and a second perceptible prompt may send a message to the user 20 that the part of the user's body is too close to the uncrewed vehicle 120. However, designs of the perceptible prompt are not limited in the present disclosure, and one of ordinary skill in the art may have different implementations thereof according to their needs.

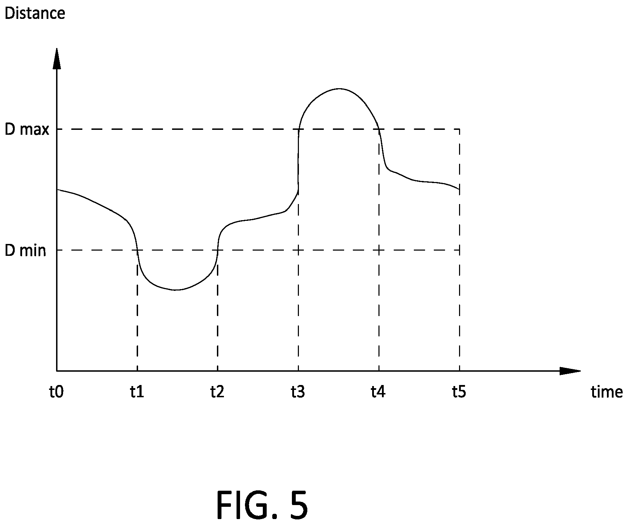

FIG. 5 is a timing diagram illustrating a distance between the uncrewed vehicle and a part of the user's body according to an example implementation of the present disclosure.

Referring to FIG. 5, a distance between an uncrewed vehicle 120 and a part of the user's body is depicted over time.

In some implementations, at time point t0, the distance between the uncrewed vehicle 120 and the part of the user's body is within a predetermined range [Dmin, Dmax], and the uncrewed vehicle 120 may start to move along the first preset trajectory, and the part of the user's body follows the uncrewed vehicle 120.

In the period t0 to t1, the part of the user's body follows the uncrewed vehicle 120 well (e.g., the body reaction of the user matches the preset reaction corresponding to the first preset trajectory).

In some implementations, at time point t1, the controller 100 may determine that the body reaction of the user 20 does not match the preset reaction corresponding the first preset trajectory because the distance between the uncrewed vehicle 120 and the part of the user's body is less than the minimum threshold Dmin. In this case, the controller 100 may control the uncrewed vehicle 120 to move away from the part of the user's body, until the controller 100 finds the distance between the uncrewed vehicle 120 and the part of the user's body is not less than the minimum threshold Dmin (e.g., at time point t2).

In the period t1 to t2, the body reaction of the user does not match the preset reaction corresponding to the first preset trajectory, and the uncrewed vehicle 120 moves away from the part of the user's body to cure the mismatch.

In some implementations, at the time point t2, the controller 100 may control the uncrewed vehicle 120 to continue the movement along the first preset trajectory, such that the part of the user's body may continue to follow the uncrewed vehicle 120 to do the training.

In the period t2 to t3, the part of the user's body follows the uncrewed vehicle 120 well (e.g., the body reaction of the user matches the preset reaction corresponding to the first preset trajectory).

In some implementations, at time point t3, the controller 100 may determine that the body reaction of the user 20 does not match the preset reaction corresponding the first preset trajectory because the distance between the uncrewed vehicle 120 and the part of the user's body is greater than the maximum threshold Dmax. In this case, the controller 100 may control the uncrewed vehicle 120 to provide the perceptible prompt including suspending the movement along the first preset trajectory, until the controller 100 finds the distance between the uncrewed vehicle 120 and the part of the user's body is not greater than the maximum threshold Dmax (e.g., at time point t4).

In the period t3 to t4, the body reaction of the user does not match the preset reaction corresponding to the first preset trajectory, and the training system 10 waits for the part of the user's body to catch up the uncrewed vehicle 120. In other words, the mismatch during the period t3 to t4 is cured by the user 20 instead of the training system 10.

In some implementations, at time point t4, the controller 100 may control the uncrewed vehicle 120 to continue the movement along the first preset trajectory (e.g., continue from the suspension point), such that the part of the user's body may continue to follow the uncrewed vehicle 120 to do the training.

In the period t4 to t5, the part of the user's body follows the uncrewed vehicle 120 well (e.g., the body reaction of the user matches the preset reaction corresponding to the first preset trajectory). The training may, for example, end at time point t5.

Instead of being guided by the training system 10, in some implementations not falling under the scope of the claims, the user 20 may further control the uncrewed vehicle 120 through a movement of a part of the user's body, such as a contraction of a specific muscle, or a force exertion of a part of the user's body. For example, the user 20 may control the uncrewed vehicle (e.g., UAV) to move toward the user 20 by beckoning and may control the uncrewed vehicle to move away from the user 20 by waving. For another example, the user 20 may control the uncrewed vehicle (e.g., UAV) to move (e.g., fly) upward by applying an upward force through the finger of the user 20, and may control the uncrewed vehicle (e.g., UAV) to move (e.g., fly) downward by applying a downward force through the finger of the user 20. For another example, the user 20 may control the uncrewed vehicle (e.g., UAV) to move (e.g., fly) upward by contracting the muscles to lift the finger(s) up, and may control the uncrewed vehicle (e.g., UAV) to move (e.g., fly) downward by contracting the muscles to lower the finger(s) down. Accordingly, not only may the user be guided by the uncrewed vehicle 120, the user 20 may also control the uncrewed vehicle 120 to move along any expected trajectories.

Compared to the traditional guidance provided by video or audio, guidance provided by the uncrewed vehicles in the present disclosure may result in a larger range of the center of pressure (COP), a more unstable COP progress, and a reduced smoothness of limb movement.

In light of the foregoing description, implementations of the training method and the training system of the present disclosure provide motion guidance in a three-dimensional real world by utilizing uncrewed vehicles, which not only brings challenges to different users as to their needs, but also involves visual feedback in functional training. Hence, a more comprehensive and effective training is achieved.

From the present disclosure, various techniques may be used for implementing the concepts described in the present application without departing from the scope of those concepts. Moreover, while the concepts have been described with specific reference to certain implementations, a person of ordinary skill in the art would recognize that changes may be made in form and detail without departing from the scope of those concepts. As such, the described implementations are to be considered in all respects as illustrative and not restrictive. It should also be understood that the present disclosure is not limited to the particular implementations described above. Still, many rearrangements, modifications, and substitutions are possible without departing from the scope of the present disclosure.

## Claims

1. A training method performed by a training system (10) comprising a plurality of uncrewed vehicles (120), the training method comprising:
controlling movements of the plurality of uncrewed vehicles (120) along a plurality of first preset trajectories respectively;
detecting a plurality of body reactions of a plurality of parts of a user's (20) body, each of the plurality of body reactions corresponding to one of the plurality of uncrewed vehicles;
determining whether the plurality of body reactions match a plurality of preset reactions corresponding to the plurality of first preset trajectories of the plurality of uncrewed vehicles; and
in a case of determining that one of the plurality of body reactions does not match the corresponding preset reaction, controlling the corresponding uncrewed vehicle (120) to provide a perceptible prompt,
wherein at least two of the plurality of first present trajectories are different.

2. The training method of claim 1, wherein controlling the corresponding uncrewed vehicle (120) to provide the perceptible prompt comprises:
suspending the movement of the corresponding uncrewed vehicle (120).

3. The training method according to claim 1 or 2, wherein in a case of determining that one of the plurality of body reactions matches the corresponding preset reaction, the method further comprises:
continuing the movement of the corresponding uncrewed vehicle (120) along one of the plurality of first preset trajectories of the corresponding uncrewed vehicle (120).

4. The training method in any one of claims 1 to 3, wherein controlling the corresponding uncrewed vehicle (120) to provide the perceptible prompt comprises:
controlling the movement of the plurality of uncrewed vehicles (120) along a plurality of second preset trajectories, wherein the plurality of second trajectories are different from the plurality of first preset trajectories.

5. The training method in any one of claims 1 to 4, wherein detecting the plurality of body reactions of the plurality of parts of the user's (20) body comprises obtaining location information of each of the plurality of parts of the user's (20) body.

6. The training method according to claim 5, wherein determining whether the plurality of body reactions match the plurality of preset reactions corresponding to the plurality of first preset trajectories comprises:
determining, based on the location information, whether a distance between one of the plurality of uncrewed vehicles (120) and one of the plurality of parts of the user's (20) body is within a predetermined range;
in a case that the distance is outside the predetermined range, determining that one of the plurality of the body reactions does not match one of the plurality of the preset reactions corresponding to one of the plurality of the first preset trajectories; and
in a case that the distance is within the predetermined range, determining that one of the plurality of the body reactions matches one of the plurality of the preset reactions corresponding to one of the plurality of the first preset trajectories.

7. The training method according to claim 5, wherein determining whether the plurality of body reactions match the plurality of preset reactions corresponding to the plurality of first preset trajectories comprises:
determining, based on the location information, whether a distance between one of the plurality of uncrewed vehicles (120) and one of the plurality of parts of the user's (20) body is greater than a maximum threshold;
in a case that the distance is greater than the maximum threshold, determining that one of the plurality of the body reactions does not match one of the plurality of the preset reactions corresponding to one of the plurality of the first preset trajectories; and
in a case that the distance is not greater than the maximum threshold, determining that one of the plurality of the body reactions matches one of the plurality of the preset reactions corresponding to one of the plurality of the first preset trajectories.

8. The training method according to claim 5, wherein determining whether the plurality of body reactions match the plurality of preset reactions corresponding to the plurality of first preset trajectories comprises:
determining, based on the location information, whether a distance between one of the plurality of uncrewed vehicles (120) and one of the plurality of parts of the user's (20) body is less than a minimum threshold;
in a case that the distance is less than the minimum threshold, determining that one of the plurality of the body reactions does not match one of the plurality of the preset reactions corresponding to one of the plurality of the first preset trajectories; and
in a case that the distance is not less than the minimum threshold, determining that one of the plurality of the body reactions matches one of the plurality of the preset reactions corresponding to one of the plurality of the first preset trajectories.

9. The training method according to any one of claims 1, 5 and 8, wherein controlling the plurality of the uncrewed vehicles (120) to provide the perceptible prompt comprises:
controlling the plurality of the uncrewed vehicles (120) to move away from the plurality of parts of the user's (20) body.

10. The training method in any one of claims 1 to 9, wherein detecting the plurality of body reactions of the plurality of parts of the user's (20) body comprises obtaining muscle strength information of the plurality of parts of the user's (20) body, and the muscle strength information comprises at least one of a strength magnitude and a strength direction.

11. A training system (10), comprising:
a plurality of uncrewed vehicles (120), each comprising:
a sensor (110) configured to detect a body reaction of a user's (20) body;
a controller (100) coupled to the uncrewed vehicle (120) and the sensor (110); and
a memory (130) coupled to the controller (100) and storing a first preset trajectory, wherein
the memory (130) further stores at least one computer-executable instruction that, when executed by the controller (100), causes the controller (100) to perform the training method as claimed in any one of claims 1 to 10.

## Patentansprüche

1. Trainingsverfahren, das von einem Trainingssystem (10) durchgeführt wird, das eine Vielzahl von unbemannten Fahrzeugen (120) umfasst, wobei das Trainingsverfahren umfasst:
Steuern der Bewegungen der Vielzahl von unbemannten Fahrzeugen (120) entlang einer Vielzahl von jeweils ersten voreingestellten Trajektorien;
Erfassen einer Vielzahl von Körperreaktionen einer Vielzahl von Teilen des Körpers eines Benutzers (20), wobei jede der Vielzahl von Körperreaktionen einem der Vielzahl von unbemannten Fahrzeugen entspricht;
Bestimmen, ob die Vielzahl von Körperreaktionen mit einer Vielzahl von voreingestellten Reaktionen übereinstimmen, die die Vielzahl von ersten voreingestellten Trajektorien der Vielzahl von unbemannten Fahrzeugen entsprechen; und
in einem Fall, in dem festgestellt wird, dass eine der Vielzahl von Körperreaktionen nicht mit der entsprechenden voreingestellten Reaktion übereinstimmt, Steuern des entsprechenden unbemannten Fahrzeugs (120), um eine wahrnehmbare Aufforderung zu liefern,
wobei mindestens zwei der Vielzahl von ersten vorhandenen Trajektorien unterschiedlich sind.

2. Trainingsverfahren nach Anspruch 1, wobei Steuern des entsprechenden unbemannten Fahrzeugs (120) zum Bereitstellen der wahrnehmbaren Aufforderung umfasst:
Unterbrechen der Bewegung des entsprechenden unbemannten Fahrzeugs (120).

3. Trainingsverfahren nach Anspruch 1 oder 2, wobei das Verfahren im Falle des Bestimmens, dass eine der Vielzahl von Körperreaktionen mit der entsprechenden vorgegebenen Reaktion übereinstimmt, weiter umfasst:
Fortsetzen der Bewegung des entsprechenden unbemannten Fahrzeugs (120) entlang einer der Vielzahl von ersten voreingestellten Trajektorien des entsprechenden unbemannten Fahrzeugs (120).

4. Trainingsverfahren nach einem der Ansprüche 1 bis 3, wobei Steuern des entsprechenden unbemannten Fahrzeugs (120) zum Bereitstellen der wahrnehmbaren Aufforderung umfasst:
Steuern der Bewegung der Vielzahl von unbemannten Fahrzeugen (120) entlang einer Vielzahl von zweiten voreingestellten Trajektorien, wobei die Vielzahl von zweiten Trajektorien von der Vielzahl von ersten voreingestellten Trajektorien verschieden ist.

5. Trainingsverfahren nach einem der Ansprüche 1 bis 4, wobei das Erfassen der Vielzahl von Körperreaktionen der Vielzahl von Körperteilen des Benutzers (20) das Erhalten von Ortsinformationen von jedem der Vielzahl von Körperteilen des Benutzers (20) umfasst.

6. Trainingsverfahren nach Anspruch 5, wobei Bestimmen, ob die Vielzahl von Körperreaktionen mit der Vielzahl von voreingestellten Reaktionen übereinstimmt, die der Vielzahl von ersten voreingestellten Trajektorien entsprechen, umfasst:
Bestimmen, basierend auf der Ortsinformation, ob ein Abstand zwischen einem der Vielzahl von unbemannten Fahrzeugen (120) und einem der Vielzahl von Körperteilen des Benutzers (20) innerhalb eines vorbestimmten Bereichs liegt;
in einem Fall, in dem der Abstand außerhalb des vorbestimmten Bereichs liegt, Bestimmen, dass eine der Vielzahl der Körperreaktionen nicht mit einer der Vielzahl der voreingestellten Reaktionen übereinstimmt, die einer der Vielzahl der ersten voreingestellten Trajektorien entspricht; und
in einem Fall, in dem der Abstand innerhalb des vorbestimmten Bereichs liegt, Bestimmen, dass eine der Vielzahl der Körperreaktionen mit einer der Vielzahl der voreingestellten Reaktionen übereinstimmt, die einer der Vielzahl der ersten voreingestellten Trajektorien entspricht.

7. Trainingsverfahren nach Anspruch 5, wobei Bestimmen, ob die Vielzahl von Körperreaktionen mit der Vielzahl von voreingestellten Reaktionen übereinstimmt, die der Vielzahl von ersten voreingestellten Trajektorien entsprechen, umfasst:
Bestimmen, basierend auf der Ortsinformation, ob ein Abstand zwischen einem der Vielzahl von unbemannten Fahrzeugen (120) und einem der Vielzahl von Körperteilen des Benutzers (20) größer als ein maximaler Schwellenwert ist;
in einem Fall, in dem der Abstand größer als der maximale Schwellenwert ist, Bestimmen, dass eine der Vielzahl der Körperreaktionen nicht mit einer der Vielzahl der voreingestellten Reaktionen übereinstimmt, die einer der Vielzahl der ersten voreingestellten Trajektorien entspricht; und
in einem Fall, in dem der Abstand nicht größer als der maximale Schwellenwert ist, Bestimmen, dass eine der Vielzahl der Körperreaktionen mit einer der Vielzahl der voreingestellten Reaktionen übereinstimmt, die einer der Vielzahl der ersten voreingestellten Trajektorien entspricht.

8. Trainingsverfahren nach Anspruch 5, wobei Bestimmen, ob die Vielzahl von Körperreaktionen mit der Vielzahl von voreingestellten Reaktionen übereinstimmt, die der Vielzahl von ersten voreingestellten Trajektorien entsprechen, umfasst:
Bestimmen, basierend auf der Ortsinformation, ob ein Abstand zwischen einem der Vielzahl von unbemannten Fahrzeugen (120) und einem der Vielzahl von Körperteilen des Benutzers (20) kleiner als ein minimaler Schwellenwert ist;
in einem Fall, in dem der Abstand kleiner als der minimale Schwellenwert ist, Bestimmen, dass eine der Vielzahl der Körperreaktionen nicht mit einer der Vielzahl der voreingestellten Reaktionen übereinstimmt, die einer der Vielzahl der ersten voreingestellten Trajektorien entspricht; und
in einem Fall, in dem der Abstand nicht kleiner als der minimale Schwellenwert ist, Bestimmen, dass eine der Vielzahl der Körperreaktionen mit einer der Vielzahl der voreingestellten Reaktionen übereinstimmt, die einer der Vielzahl der ersten voreingestellten Trajektorien entspricht.

9. Trainingsverfahren nach einem der Ansprüche 1, 5 und 8, wobei Steuern der Vielzahl der unbemannten Fahrzeuge (120) zur Bereitstellung der wahrnehmbaren Aufforderung umfasst:
Steuern der Vielzahl der unbemannten Fahrzeuge (120), um sich von der Vielzahl von Körperteilen des Benutzers (20) wegzubewegen.

10. Trainingsverfahren nach einem der Ansprüche 1 bis 9, wobei das Erfassen der Vielzahl von Körperreaktionen der Vielzahl von Teilen des Körpers des Benutzers (20) das Erhalten von Muskelkraftinformationen der Vielzahl von Teilen des Körpers des Benutzers (20) umfasst, und die Muskelkraftinformationen mindestens eines von einer Kraftgröße und einer Kraftrichtung umfassen.

11. Trainingssystem (10), umfassend:
eine Vielzahl von unbemannten Fahrzeugen (120), jeweils umfassend:
einen Sensor (110), der konfiguriert ist, um eine Körperreaktion des Körpers eines Benutzers (20) zu erfassen;
eine Steuereinrichtung (100), die mit dem unbemannten Fahrzeug (120) und dem Sensor (110) verbunden ist; und
einen Speicher (130), der mit der Steuereinrichtung (100) gekoppelt ist und eine erste voreingestellte Trajektorie speichert, wobei
der Speicher (130) weiter mindestens einen computerausführbaren Befehl speichert, der, wenn er von der Steuereinrichtung (100) ausgeführt wird, die Steuereinrichtung (100) veranlasst, das in einem der Ansprüche 1 bis 10 beanspruchte Trainingsverfahren durchzuführen.

## Revendications

1. Procédé d'entrainement réalisé par un système d'entrainement (10) comprenant une pluralité de véhicules sans équipage (120), le procédé d'entrainement comprenant :
la commande de mouvements de la pluralité de véhicules sans équipage (120) le long d'une pluralité de premières trajectoires prédéfinies respectivement ;
la détection d'une pluralité de réactions corporelles provenant d'une pluralité de parties du corps d'un utilisateur (20), chacune de la pluralité des réactions corporelles correspondant à l'un de la pluralité de véhicules sans équipage ;
la détermination du fait que la pluralité de réactions corporelles correspondent à une pluralité de réactions prédéfinies correspondant à la pluralité de premières trajectoires prédéfinies de la pluralité de véhicules sans équipage ; et
dans le cas de la détermination du fait que l'une de la pluralité de réactions corporelles ne correspond pas à la réaction prédéfinie correspondante, la commande du véhicule sans équipage (120) correspondant pour fournir une invite perceptible,
dans lequel au moins deux de la pluralité de premières trajectoires présentes sont différentes.

2. Procédé d'entraînement selon la revendication 1, dans lequel la commande du véhicule sans équipage (120) correspondant pour fournir l'invite perceptible comprend :
la suspension du mouvement du véhicule sans équipage (120) correspondant.

3. Procédé d'entraînement selon la revendication 1 ou la revendication 2, dans lequel dans un cas où il est déterminé que l'une de la pluralité de réactions corporelles correspond à la réaction prédéfinie correspondante, le procédé comprend en outre :
la poursuite du déplacement du véhicule sans équipage (120) correspondant le long de l'une de la pluralité de premières trajectoires prédéfinies du véhicule sans équipage (120) correspondant.

4. Procédé d'entraînement selon l'une quelconque des revendications 1 à 3, dans lequel la commande du véhicule sans équipage (120) correspondant pour fournir l'invite perceptible comprend :
la commande du mouvement de la pluralité de véhicules sans équipage (120) le long d'une pluralité de secondes trajectoires prédéfinies, dans lequel la pluralité de secondes trajectoires sont différentes de la pluralité de premières trajectoires prédéfinies.

5. Procédé d'entraînement selon l'une quelconque des revendications 1 à 4, dans lequel la détection de la pluralité de réactions corporelles de la pluralité de parties du corps de l'utilisateur (20) consiste à obtenir des informations d'emplacement pour chacune de la pluralité de parties du corps de l'utilisateur (20).

6. Procédé d'entraînement selon la revendication 5, dans lequel la détermination du fait que la pluralité de réactions corporelles correspondent ou non à la pluralité de réactions prédéfinies correspondant à la pluralité de premières trajectoires prédéfinies comprend :
la détermination, sur la base des informations d'emplacement, du fait qu'une distance entre l'un de la pluralité de véhicules sans équipage (120) et l'une de la pluralité de parties du corps de l'utilisateur (20) est ou non dans une plage prédéterminée ;
dans un cas où la distance est en dehors de la plage prédéterminée, la détermination du fait que l'une de la pluralité des réactions corporelles ne correspond pas à l'une de la pluralité des réactions prédéfinies correspondant à l'une de la pluralité des premières trajectoires prédéfinies ; et
dans un cas où la distance est dans la plage prédéterminée, la détermination du fait que l'une de la pluralité des réactions corporelles correspond à l'une de la pluralité des réactions prédéfinies correspondant à l'une de la pluralité des premières trajectoires prédéfinies.

7. Procédé d'entraînement selon la revendication 5, dans lequel la détermination du fait que la pluralité de réactions corporelles correspondent ou non à la pluralité de réactions prédéfinies correspondant à la pluralité de premières trajectoires prédéfinies comprend :
la détermination, sur la base des informations d'emplacement, du fait qu'une distance entre l'un de la pluralité de véhicules sans équipage (120) et l'une de la pluralité de parties du corps de l'utilisateur (20) est supérieure ou non à un seuil maximal ;
dans un cas où la distance est supérieure au seuil maximal, la détermination du fait que l'une de la pluralité des réactions corporelles ne correspond pas à l'une de la pluralité des réactions prédéfinies correspondant à l'une de la pluralité des premières trajectoires prédéfinies ; et
dans un cas où la distance n'est pas supérieure au seuil maximal, la détermination du fait que l'une de la pluralité des réactions corporelles correspond à l'une de la pluralité des réactions prédéfinies correspondant à l'une de la pluralité des premières trajectoires prédéfinies.

8. Procédé d'entraînement selon la revendication 5, dans lequel la détermination du fait que la pluralité de réactions corporelles correspondent ou non à la pluralité de réactions prédéfinies correspondant à la pluralité de premières trajectoires prédéfinies comprend :
la détermination, sur la base des informations d'emplacement, du fait qu'une distance entre l'un de la pluralité de véhicules sans équipage (120) et l'une de la pluralité de parties du corps de l'utilisateur (20) est inférieure ou non à un seuil minimal ;
dans un cas où la distance est inférieure au seuil minimal, la détermination du fait que l'une de la pluralité des réactions corporelles ne correspond pas à l'une de la pluralité des réactions prédéfinies correspondant à l'une de la pluralité des premières trajectoires prédéfinies ; et
dans un cas où la distance n'est pas inférieure au seuil minimal, la détermination du fait que l'une de la pluralité des réactions corporelles correspond à l'une de la pluralité des réactions prédéfinies correspondant à l'une de la pluralité des premières trajectoires prédéfinies.

9. Procédé d'entraînement selon l'une quelconque des revendications 1, 5 et 8, dans lequel la commande de la pluralité des véhicules sans équipage (120) pour fournir l'invite perceptible comprend :
la commande de la pluralité des véhicules sans équipage (120) pour s'éloigner de la pluralité de parties du corps de l'utilisateur (20).

10. Procédé d'entraînement selon l'une quelconque des revendications 1 à 9, dans lequel la détection de la pluralité de réactions corporelles de la pluralité de parties du corps de l'utilisateur (20) comprend l'obtention d'informations de force musculaire de la pluralité de parties du corps de l'utilisateur (20), et les informations de force musculaire comprennent au moins l'une parmi une magnitude de force et une direction de force.

11. Système d'entraînement (10) comprenant :
une pluralité de véhicules sans équipage (120) comprenant chacun :
un capteur (110) configuré pour détecter une réaction corporelle du corps d'un utilisateur (20) ;
un dispositif de commande (100) couplé au véhicule sans équipage (120) et au capteur (110) ; et
une mémoire (130) couplée au dispositif de commande (100) et stockant une première trajectoire prédéfinie, dans lequel
la mémoire (130) stocke en outre au moins une instruction exécutable par ordinateur qui, lorsqu'elle est exécutée par le dispositif de commande (100), amène le dispositif de commande (100) à réaliser le procédé d'entraînement tel que revendiqué dans l'une quelconque des revendications 1 à 10.
